(19) Europäisches Patentamt

European Patent Office

Office européen des brevets

(11) **EP 0 675 104 B1**

(12) **EUROPÄISCHE PATENTSCHRIFT**

(45) Veröffentlichungstag und Bekanntmachung des Hinweises auf die Patenterteilung:
**09.06.1999 Patentblatt 1999/23**

(51) Int. Cl.$^6$: **C07C 205/12**, C07C 201/08

(21) Anmeldenummer: **95103944.5**

(22) Anmeldetag: **17.03.1995**

(54) **Verfahren zur adiabatischen Herstellung von Mononitrohalogenbenzolen**

Process for adiabatic preparation of mononitrohalogenbenzenes

Procédé pour la préparation adiabatique de mononitrohalogénobenzènes

(84) Benannte Vertragsstaaten:
**BE DE FR GB IT NL PT SE**

(30) Priorität: **30.03.1994 DE 4411064**

(43) Veröffentlichungstag der Anmeldung:
**04.10.1995 Patentblatt 1995/40**

(73) Patentinhaber: **BAYER AG**
**51368 Leverkusen (DE)**

(72) Erfinder:
 • **Blank, Heinz Ulrich, Dr.**
  **D-51519 Odenthal (DE)**
 • **Judat, Helmut, Dr.**
  **D-40764 Langenfeld (DE)**
 • **König, Bernd-Michael, Dr.**
  **D-51467 Bergisch Gladbach (DE)**

(56) Entgegenhaltungen:
EP-A- 0 551 144      US-A- 2 256 999
US-A- 4 021 498      US-A- 4 453 027

## Beschreibung

Die vorliegende Erfindung betrifft ein Verfahren zur hochselektiven, abfallsäurefreien Herstellung von Mononitrohalogenbenzolen unter Ausnutzung der Reaktionswärme.

Mononitrohalogenbenzole sind wichtige Zwischenprodukte zur Herstellung von Kunststoffen, Farbstoffen und Hilfsmitteln.

Mononitrohalogenbenzole werden technisch durch isotherme Nitrierung von Halogenbenzolen bei relativ niedrigen Temperaturen (60 bis 90°C) hergestellt. Dabei fallen große Mengen verunreinigter Abfallsäure an, die kostenintensiv entsorgt oder aufgearbeitet werden müssen. Nachteilig bei diesen Verfahren ist, daß eine erhebliche Reaktionswärme abgeführt werden muß und diese Energie auf einem niedrigen Niveau anfällt, so daß sie nicht verwertet werden kann.

Ein weiterer Nachteil besteht darin, daß zusätzliche Energie zur Aufkonzentrierung der kalten Gebrauchtsäure aufgewendet werden muß. Schwierigkeiten bereitet zusätzlich die Trennung von organischer und anorganischer Phase nach der Nitrierung. Restliches organisches Material muß aus der Gebrauchtsäure durch Extraktion entfernt werden.

Um den Anfall von Abfallsäure zu vermeiden, sind Verfahren anzustreben, die eine integrierte Schwefelsäureaufkonzentrierung unter Ausnutzung der Reaktionswärme beinhalten. Dies bedingt eine Kreislaufsäure, in der sich Nebenprodukte nicht anreichern dürfen und hohe Reaktionstemperaturen, um die Schwefelsäure technisch kostengünstig aufkonzentrieren zu können.

Für Benzol ist die adiabatische Mononitrierung in einer Reihe von Patentschriften beschrieben (US 2 256 999, US 4 021 498, US 4 091 042, US 4 973 770, EP 436 443). Die oben genannten energetischen Nachteile bei der isothermen Nitrierung von Halogenbenzolen entfallen bei der adiabatischen Benzol-Nitrierung, da deren Reaktionswärme auf hohem Niveau (Temperatur der Gebrauchtsäure >100°C) anfällt und zur Aufkonzentrierung der Säure benutzt werden kann. Die Ausdehnung dieser Verfahrensweise auf die Mononitrierung von Halogenbenzolen wird zwar in US 2 256 999, US 4 021 498, US 4 973 770 und EP 436 443 erwähnt, ist aber dort nicht in einem Beispiel beschrieben worden. Daher mußte angenommen werden, daß die adiabatische Nitrierung anderer Aromaten als Benzol nach diesen Patentschriften rein spekulativen Charakter hat. Die Temperatur von 100°C nach US 4 973 770 liegt beispielsweise oberhalb der aus der isothermen Nitrierung von Halogenbenzolen bekannten (s.o.). Am Beispiel von US 4 021 498 sei darauf verwiesen, daß zur Reaktionsführung starkes Rühren für erforderlich gehalten wird (Kol. 3, Z. 60 und Kol. 4, Z. 54 von US '498); selbst in Rohrreaktoren werden Rührer benötigt (Kol. 4, z. 41 von US '498).

Bei dem in US 4 973 770 beschriebenen Verfahren wird ferner auf eine einmalige Verdüsung und Feinverteilung des Benzols abgestellt mit Hilfe derer der gesamte Reaktionsablauf bewältigt werden soll. Um die Feinverteilung möglichst lange aufrechtzuerhalten, muß die Koaleszenz der verdüsten Partikel untereinander und an der Wand vermieden werden; zur Vermeidung der Wandberührung wird ein Reaktor mit großem Durchmesser, verglichen mit dem Düsendurchmesser, eingesetzt, der eine starke Rückvermischung zuläßt: Gemäß Ausführungsbeispiel von US 4 973 770 in Verbindung mit Fig. 1 wird Benzol durch eine Sprühdüse mit 0,5 mm Durchmesser in einen Reaktor mit einem Durchmesser von 75 mm eingeleitet. Bei einer Gesamtlänge des Reaktors von 430 mm wird die Mischsäure in einem Abstand von 150 mm von der Düse zugegeben, wo die Energie des Düsenstrahls bereits weitgehend für die Rückvermischung verbraucht ist. Die Vermischung der zugegebenen Mischsäure mit dem eingesprühten Benzol geschieht auf einem niedrigen Energieniveau und mit einer geringeren Intensität.

Auch der Anspruch von EP 436 443, demzufolge mit Nitronium-Ionen enthaltenden Mischsäuren mit molaren Zusammensetzungen gearbeitet wird, die der Fig. 6 in EP 436 443 zu entnehmen sind, ist außerhalb dessen, was dem Fachmann geläufig ist. So bezeichnet der in Fig. 6 herausgestellte Punkt D in Verbindung mit S. 9, Z. 10 bis 12 von EP 436 443 eine Säure der Zusammensetzung von 72,02 Mol-% $H_2SO_4$, 2,99 Mol-% $HNO_3$ und 24,99 Mol-% $H_2O$, das entspricht 91,71 Gew.-% $H_2SO_4$, 2,45 Gew.-% $HNO_3$ und 5,84 Gew.-% $H_2O$. Eine derart starke Säure ist für ein technisches wirtschaftliches Verfahren zur adiabatischen Herstellung von Mononitrohalogenbenzolen ungeeignet. Darüberhinaus ist eine Kreisführung für die in diesem Verfahren entstehende Abfallsäure technisch nicht vertretbar, weil die notwendige Aufkonzentrierung auf weit über 90 Gew.-% zu aufwendig ist. Dieses Verfahren ist daher wirtschaftlich nicht sinnvoll und vermag dem Fachmann keinen Hinweis zur Lösung der vorliegenden Aufgabe zu vermitteln.

Ein adiabatisches Nitrierverfahren zur Herstellung von Mononitrohalogenbenzolen wird in US 4 453 027 beansprucht. Die dort beschriebenen Bedingungen sind für eine sinnvolle technische Durchführung jedoch ungeeignet. Die verwendete Nitriersäure enthält 11,2 Gew.-% $HNO_3$, 68,5 Gew.-% $H_2SO_4$ und 20,3 Gew.-% $H_2O$. Für die ablaufende Gebrauchtsäure wird eine Temperatur von 100 bis 110°C angegeben. Bei adiabatischer Durchführung beträgt mit einer solchen $HNO_3$-Konzentration die Temperaturerhöhung jedoch während der Reaktion ca. 100°C, sodaß die Reaktion bei 0 bis 10°C begonnen werden muß und die einzusetzenden Reaktionspartner bei dieser Temperatur zur Verfügung stehen müssen. Die ablaufende Gebrauchtsäure (Absäure) muß bei Verwendung von 60 %iger $HNO_3$ auf 84,1 Gew.-% $HSO_4$, bei Verwendung von 98 %iger $HNO_3$ immerhin noch auf 77,3 Gew.-% $H_2SO_4$ aufkonzentriert werden. Wird die Reaktionswärme dazu genutzt, kühlt sich die Absäure dabei auf ca. 0°C ab. Ein solches Verfahren ist technisch und wirtschaftlich nicht mehr sinnvoll, da Kühlsole und Spezialapparate eingesetzt werden müssen.

Halogenbenzole lassen sich jedoch mit hoher Selektivität adiabatisch bei hohen Reaktionstemperaturen mononitrieren, wenn die im folgenden beschriebenen Schritte und Bedingungen angewandt werden.

Die Erfindung betrifft ein Verfahren zur kontinuierlichen Herstellung von Mononitrohalogenbenzolen durch Umsetzung von Halogenbenzolen mit einem $HNO_3/H_2SO_4/H_2O$-Gemisch unter Bildung im wesentlichen der Mononitrohalogenbenzole und Reaktionswasser, gekennzeichnet durch die Schritte

a) Einspeisung der Reaktionsteilnehmer Halogenbenzol, $HNO_3$, $H_2SO_4$ und $H_2O$ in beliebiger Reihenfolge in einen mit Mischungsorganen ausgestatteten Reaktor, wobei

a1) die Menge an $HNO_3$ 1 bis 8 Gew.-%, die Menge an $H_2SO_4$ 56,5 bis 84,5 Gew.-% und die Menge an $H_2O$ den Rest zu 100 Gew.-% beträgt und 100 Gew.-% die Summe von $HNO_3$ + $H_2SO_4$ + $H_2O$ darstellen,

a2) das $H_2O$ als solches, als Verdünnungs-$H_2O$ der $HNO_3$, als Verdünnungs-$H_2O$ der $H_2SO_4$ oder in mehreren der genannten Formen eingesetzt wird, und

a3) das molare Verhältnis von Halogenbenzol zu $HNO_3$ 0,9 bis 1,5 beträgt,

b) rasche und intensive Vermischung der Gesamtmenge der Reaktionsteilnehmer, wozu eine Mischenergie von 1 bis 40 Watt pro Liter des gesamten Reaktionsgemisches, bevorzugt 3 bis 30 W/l, angewandt wird,

c) Durchführung der Umsetzung unter adiabatische Bedingungen in Reaktoren, die die Rückvermischung der Reaktionsteilnehmer weitgehend verhindern und in denen die Reaktionsteilnehmer beim Durchströmen mindestens zweimal redispergiert werden, wobei die Reaktionsteilnehmer mit solchen Temperaturen eingespeist werden, daß die Vermischung im Bereich von 60 bis 160°C erfolgt und die Temperatur am Ende der Reaktion 180°C nicht übersteigt,

d) Trennung des Reaktionsgemisches nach Durchführung der Reaktion in eine organische und eine anorganische Phase und

e) destillative Aufarbeitung der weitgehend $HNO_3$-freien anorganischen Phase unter Entfernung von Wasser.

Halogenbenzole im Sinne der Erfindung sind Chlorbenzol, o-, m-, p-Dichlorbenzol, o-, m-, p-Chlortoluol und Brombenzol, bevorzugt Chlorbenzol und o-, m-, p-Dichlorbenzol, besonders bevorzugt Chlorbenzol und o-Dichlorbenzol.

Das erfindungsgemäße Verfahren kann kontinuierlich oder diskontinuierlich, bevorzugt kontinuierlich durchgeführt werden.

Zur kontinuierlichen Durchführung kann beispielsweise so vorgegangen werden: Die Gesamtmenge der Reaktionsteilnehmer wird mit Hilfe von Mischorganen rasch vermischt und als Mischung in einen Reaktor eingespeist. Die Mischungszeit bei kontinuierlicher Durchführung beträgt in der Regel weniger als 3 sek, beispielsweise 1 msek bis 2,99 sek, bevorzugt 1 msek bis 2 sek. Der Reaktor ist gegebenenfalls isoliert, verhindert weitgehend die Rückvermischung und wird adiabatisch betrieben. Zur weitgehenden Verhinderung der Rückvermischung ist der Reaktor unterteilt oder besteht aus mehreren Kammern oder Einheiten; an den Übergängen zwischen den Reaktorteilen wird das Reaktionsgemisch redispergiert. Das ausreagierte Gemisch läuft ab und wird in einem Trenngefäß getrennt; die Trennung erfolgt rasch. Die organische Phase wird wie üblich, z.B. durch Wäsche und Destillation, aufgearbeitet oder sofort einer Zweitnitrierung zugeführt. Im allgemeinen, insbesondere bei einem Überschuß an Halogenbenzol, ist die abgetrennte anorganische Phase praktisch frei von Salpetersäure. Sollte dies, insbesondere bei einem Überschuß an Salpetersäure, nicht der Fall sein, kann restliche Salpetersäure in einem Nachreaktor unter Zusatz von weiterem Halogenbenzol im Sinne einer Reaktiv-Extraktion verbraucht werden. Die von der Salpetersäure weitgehend befreite anorganische Säurephase wird bevorzugt unter Ausnutzung der aufgenommenen Reaktionswärme und unter vermindertem Druck einer Flash-Verdampfung zugeführt. Hierbei wird Wasser aus der Säure entfernt und die Säure dabei in bevorzugter Weise auf die Eingangskonzentration und die Eingangstemperatur für Schritt a) gebracht. Durch diese Rückführung der aufgearbeiteten anorganischen Phase ($H_2SO_4$, $H_2O$) in den Prozeß ergibt sich eine Kreislauffahrweise für die $H_2SO_4$; es kann sinnvoll sein, einen geringen Teil dieser $H_2SO_4$ auszuschleusen, um etwaige Verunreinigungen auf einem niedrigen Niveau zu lassen. Für den Fall, daß die anorganische Phase noch Halogenbenzol, Nitrohalogenbenzol und evtl. organische Nebenprodukte enthält, kann es sinnvoll sein, die anorganische Phase vor der Flash-Verdampfung zur Entfernung der Organika zu strippen. Das danach als Flash-Kondensat erhaltene Wasser ist dann von höherer Reinheit, und seine Entsorgung ist einfacher. Selbstverständlich kann auch das Flash-Kondensat durch Strippen von Organika befreit werden, wobei analog ein restliches Flash-Kondensat von höherer Reinheit zurückbleibt. Die bei der Nachreak-

tion der $HNO_3$ mit weiteren Halogenbenzol sowie beim Strippen anfallenden Organika können dem Prozeß an geeigneter Stelle zugesetzt werden (Halogenbenzol, (Di)Nitrohalogenbenzol) oder werden ausgeschleust und entsorgt (Verunreinigungen, Nebenprodukte).

Die Reaktionsteilnehmer können gemeinsam, jedoch auch einzeln oder als Gemische zweier oder dreier von ihnen gleichzeitig oder sukzessive dem mit Mischungsorganen ausgestatteten Reaktor zugeführt werden. Die Vermischung der Einsatzstoffe kann beispielsweise so stattfinden, daß man Halogenbenzol und Salpetersäure als zwei separate Ströme gleichzeitig oder sukzessive der aufkonzentrierten, gebrauchten Schwefelsäure zusetzt, wobei die Salpetersäure durch Wasser und/oder Schwefelsäure und Wasser verdünnt sein kann. Das Halogenbenzol kann auch mit Wasser und Schwefelsäure vorvermischt und die resultierende Emulsion mit Salpetersäure, die mit Schwefelsäure und/oder Wasser vermischt sein kann, weiter rasch und intensiv vermischt werden. Weiterhin kann auch das Halogenbenzol mit einer Mischsäure aus $H_2SO_4$, $HNO_3$ und $H_2O$ intensiv vermischt werden. Noch weitere Varianten der Zuführung der Reaktionsteilnehmer, ihrer intensive Vermischung und erfindungsgemäßen Weiterbehandlung sind für den Fachmann leicht erkennbar. Dazu sind die in der Technik bekannten Mischorgane geeignet, z.B.: 1. Statische Mischer, 2. Pumpen, 3. Düsen, 4. Rührer oder Kombinationen hiervon.

Für das Gelingen der Reaktion ist es von geringerer Bedeutung, in welcher Reihenfolge und Zusammensetzung die Reaktionsteilnehmer Salpetersäure und Halogenbenzole sowie Schwefelsäure und Wasser miteinander vermischt werden, solange das Reaktionsgemisch nach der Gesamtvermischung die erfindungsgemäße Zusammensetzung besitzt und die Vermischung in der erfindungsgemäßen Intensität und bei kontinuierlich durchgeführter Reaktion weitgehend rückvermischungsfrei stattfindet.

Die Intensität der Vermischung kann bei der diskontinuierlichen Fahrweise neben dem hohen Energieeintrag auch durch die kurze Zugabezeit der Reaktionsteilnehmer gekennzeichnet werden, die 0,001-15 %, bevorzugt 0,001-3 % der Zeit beträgt, die für den Ablauf der Reaktion zwischen dem Halogenbenzol und $HNO_3$ erforderlich ist. Damit ist die Durchführung des erfindungsgemäßen Verfahrens absatzweise in einem Rührkessel möglich.

Der Einspeisung und intensiven Vermischung der Reaktionsteilnehmer folgen bei kontinuierlicher Durchführung mindestens zwei Dispergierungen. Hierzu sind im Reaktor Lochbleche, Schlitzbleche, Prallbleche, Rührer oder ähnliche, dem Fachmann für diesen Zweck bekannte Einbauten bzw. Organe vorhanden.

Als kontinuierlich betriebene Reaktoren für das erfindungsgemäße Verfahren seien beispielsweise genannt: Rohrreaktoren mit Einbauten zur Redisperigerung, wie Strömungsbrechern, Umlenkblechen, Statikmischern, Rührern und ähnlichen; stark gerührte Kessel in Kaskadenanordnung; Schlaufenreaktoren mit Einbauten wie oben; Kombinationen mehrerer der genannten Apparatate; weitere gleichwirkende Reaktoren, wie Kammerreaktoren mit Rührern in jeder Kammer. In bevorzugter Weise werden Rohrreaktoren mit Einbauten eingesetzt. Die Einbauten sind bevorzugt Lochbleche. Alle Einbauten stellen Unterteilungen der Gesamtapparatur dar, die gleichermaßen der Redispergierung und der weitgehenden Verhinderung der Rückvermischung dienen.

Nach dem intensiven Vermischen, nach jeder Dispergierung und nach dem Durchströmen einer gewissen Teillänge der Reaktors wird ein Koaleszieren der Dispersionströpfchen beobachtet, was durch Redispergierung rückgängig gemacht wird. Die Zahl der Dispergierungsvorgänge beträgt erfindungsgemäß 2-50, bevorzugt 3-30, besonders bevorzugt 4-20. Zur Überwindung der dabei auftretenden Druckverluste wird mit den Reaktionsteilnehmern eine Mischenergie pro Liter des gesamten Reaktionsgemisches von 1-40 Watt/Liter, bevorzugt 3-30 W/l, in das Reaktionssystem gegeben..

Die Vermischung der Reaktionsteilnehmer erfolgt im Bereich von 60 bis 160°C, bevorzugt 70 bis 140°C, besonders bevorzugt 80 bis 120°C. Es werden adiabatische Reaktionsbedingungen eingehalten. Die Endtemperatur ist abhängig von der Höhe der Mischungstemperatur, von den Mengenverhältnissen der Reaktionsteilnehmer und vom Umsatz; sie übersteigt im allgemeinen 180°C nicht, meistens nicht 160°C.

Der Gehalt an zugesetzter Salpetersäure im Reaktionsgemisch zum Zeitpunkt der Vermischung beträgt, bezogen auf die Summe von Salpetersäure, Schwefelsäure und Wasser, 1 bis 8 Gew.-%, bevorzugt 2 bis 6 Gew.-%, besonders bevorzugt 2,5 bis 5 Gew.-%. Salpetersäure kann in hochkonzentrierter oder in azeotrop siedender Form, beispielsweise als 60-98 Gew.-%ige $HNO_3$, bevorzugt aber in Form der ca. 60-65 Gew.-% aufweisenden und billig verfügbaren "Schwachsäure" eingesetzt werden.

Der Gehalt an Schwefelsäure im Reaktionsgemisch zum Zeitpunkt der Vermischung beträgt, bezogen auf die Summe von Salpetersäure, Schwefelsäure und Wasser, 56,5 bis 84,5 Gew.-%, bevorzugt 65 bis 79 Gew.-%, besonders bevorzugt 67,5 bis 77 Gew.-%.

Der Rest zu 100 Gew.-% ist $H_2O$. Dies kann als solches, als Verdünnungs-$H_2O$ der $H_2SO_4$, als Verdünnungs-$H_2O$ der $HNO_3$ oder in mehreren der genannten Formen eingesetzt werden. In bevorzugter Form liegt $H_2O$ als Verdünnungs-$H_2O$ sowohl der $H_2SO_4$ als auch der $HNO_3$ vor.

Da die Nitrierstärke bei wechselnden Salpetersäuregehalten in der Nitriersäure abhängig ist vom Verhältnis von Schwefelsäure zu Wasser, wird sie anhand der Schwefelsäurekonzentration der ablaufenden und weitgehend salpetersäurefreien Gebrauchtsäure bestimmt und gegebenenfalls nachreguliert. Diese $H_2SO_4$-Konzentration der Gebrauchtsäure soll erfindungsgemäß 60 bis 85 Gew.-%, bevorzugt 68 bis 80 Gew.-%, besonders bevorzugt 70 bis 78 Gew.-% betragen. Zum Wiedereinsatz wird die ablaufende Schwefelsäure um 0,6 bis 7,5 Prozentpunkte, in vielen Fällen um 1,7 bis 4,2 Prozentpunkte aufkonzentriert,wobei Wasser (Reaktionswasser, gegebenenfalls Verdünnungswasser) destillativ ausgeschleust wird. Hierzu wird in bevorzugter Weise die von der ablaufenden $H_2SO_4$ aufgenommene Reaktionswärme ausgenutzt und unter vermindertem Druck, etwa bei 40 bis 150 mbar, bevorzugt bei 40 bis 120 mbar, besonders bevorzugt bei 50-100 mbar, gearbeitet. Beispielsweise kann dies in Form einer Flash-Verdampfung durchgeführt werden. Die dabei gewonnene $H_2SO_4$ ist zum Einsatz in Schritt a) geeignet. In bevorzugter Weise wird die destillative Ausschleusung von Wasser so durchgeführt, daß die Temperatur und Konzentration der aufkonzentrierten $H_2SO_4$ unmittelbar den in Schritt a) geforderten Werten entspricht. Eine solche Nutzung der Reaktionswärme macht das erfindungsgemäße Verfahren wirtschaftlicher als die bekannten Verfahren zur Herstellung von Nitrohalogenbenzolen.

Mögliche Ausführungsformen bezüglich der Mischsäuren mit wechselnden Zusammensetzungen, ablaufender $H_2SO_4$-Konzentration, Temperaturverlauf und Druck der Flash-Verdampfung sowie Aufkonzentration der $H_2SO_4$ seien beispielhaft wie folgt zusammengestellt (Fälle a und c: Chlorbenzol; Fall b: o-Dichlorbenzol):

| | a | b | c |
|---|---|---|---|
| Mischsäure | | | |
| $HNO_3$ (Gew.-%) | 3,00 | 3,00 | 5,00 |
| $H_2SO_4$ (Gew.-%) | 68,50 | 74,37 | 67,50 |
| $H_2O$ (Gew.-%) | 28,50 | 22,63 | 27,50 |
| Stärke der eingesetzten Säuren | | | |
| $HNO_3$ (Gew.-%) | 60,00 | 60,00 | 60,00 |
| $H_2SO_4$ (Gew.-%) | 72,11 | 78,28 | 73,64 |
| ablaufende $H_2SO_4$ (Gew.-%) | 70,00 | 76,00 | 70,00 |
| Mischungstemperatur (°C) | 110 | 110 | 100 |
| Endtemperatur (ca.°C) | 140 | 140 | 150 |
| Druck bei Flash-Verdampfung (ca. mbar) | 95 | 48 | 50 |

Das molare Verhältnis von Halogenbenzol zu $HNO_3$ beträgt allgemein 0,9 bis 1,5. Um die Bildung unerwünschter Dinitrohalogenbenzole zu minimieren, beträgt das Molverhältnis von Halogenbenzol zu Salpetersäure bevorzugt 1,0 bis 1,5, besonders bevorzugt 1,01 bis 1,3, ganz besonders bevorzugt 1,05 bis 1,2. Für den Fall, daß die erfindungsgemäß erhältlichen Nitrohalogenbenzole der Dinitrierung zugeführt werden sollen, sind aber auch weitere Bereiche, z.B. 0,9 bis 1,2 Mol, bevorzugt 0,9 bis 1,05 Mol, besonders bevorzugt 0,95 bis 1 Mol Halogenbenzol pro Mol Salpetersäure zulässig.

Die Reaktion des erfindungsgemäßen Verfahrens lautet formelmäßig:

$$C_6H_5\text{-Hal} + HNO_3 \rightarrow O_2N\text{-}C_6H_4\text{-Hal} + H_2O.$$

Somit werden Halogenbenzol und $HNO_3$ in das Verfahren eingeführt und Mononitrohalogenbenzol und $H_2O$ ausgeschleust, während das beschriebene $H_2SO_4/H_2O$-Gemisch das Reaktionsmedium darstellt. Da bei der technischen Realisierung vorteilhäfterweise verdünnte Salpetersäuren eingesetzt werden, muß zusätzlich zum Reaktionswasser auch noch Verdünnungs-$H_2O$ der $HNO_3$ ausgeschleust werden.

Die bei der Trennung des Reaktionsgemisches anfallende organische Phase kann auf reines Mononitrohalogenbenzol aufgearbeitet werden oder der Dinitrohalogenbenzol-Herstellung zugeführt werden. Im ersteren Fall wird man, wie oben beschrieben, mindestens molare Mengen an Halogenbenzol oder einen geringen molaren Überschuß einsetzen, um sowohl die $HNO_3$ zu verbrauchen, als auch die Zweitnitrierung zu unterdrücken; ein etwaiger Halogenbenzolüberschuß wird aus der organischen Phase abdestilliert. Zuvor kann die organische Phase gewaschen werden, um wasser-, säure- oder alkalilösliche Verunreinigungen abzutrennen, wie anorganische und organische Säuren und phenolische Verunreinigungen. Die Bildung von Oxidationsprodukten (Phenolkörper, Oxidation der $CH_3$-Gruppe) ist stark unterdruckt. Ebenso ist die Bildung von Dinitrohalogenbenzolen stark unterdrückt. Diese Dinitrohalogenbenzole stören jedoch dann nicht, wenn ohnehin eine Zweitnitrierung vorgesehen ist; daher darf in solchen Fällen auch mit einem geringen Halogenbenzolunterschuß gearbeitet werden.

Als Modell für einen rückvermischungsfreien technischen Reaktor und zur Darstellung der diskontinuierlichen Durchführung kann im Labor ein Batch-Ansatz in einem stark gerührten, wärmeisolierten Rührkolben, z.B. in einem sogenannten Sulfierbecher, der mit Stromstörern versehen ist, dienen. Dabei werden Halogenbenzol, Schwefelsäure und Wasser z.B. bei 110°C vorgelegt und mit der auf die erfindungsgemäße Einsatztemperatur erwärmten Salpetersäure, die durch Wasser und/oder Schwefelsäure verdünnt sein kann, in 1 bis 2 Sekunden versetzt. Nach der Dosierung läßt man die Reaktion adiabatisch ablaufen. Die Endtemperatur wird dabei in ca. 70 bis 100 Sekunden erreicht (Beispiele 1 bis 3). Alternativ kann auch die Gesamtmenge an $HNO_3$, $H_2SO_4$ und $H_2O$ vorgelegt und mit Halogenbenzol versetzt werden; noch weitere Dosiervarianten sind für den Fachmann leicht erkennbar. Der Inhalt des Reaktionsgefäßes entspricht dabei im zeitlichen Verlauf einem Volumenteil in der axialen Bewegung durch einen Rohrreaktor mit Pfropfenströmung. Was im Batch-Ansatz zeitlich nacheinander geschieht, läuft etwa in einem Rohrreaktor örtlich hintereinander ab.

Bei dieser Durchführung des erfindungsgemäßen Verfahrens im Labor wird im Rahmen der analytischen Schwankungsbreiten ein Gemisch von Mononitrohalogenbenzolen erhalten, das weniger als 0,01 % an dinitrierten Verbindungen enthält.

Die Ausbeute an Mononitrohalogenbenzolen, bezogen auf eingesetzte Salpetersäure, beträgt >95 % der Theorie, vielfach >97 % und bei kontinuierlicher Durchführung >98 %.

Nach Erreichen der Reaktionstemperatur wird der Rührer angehalten. Die Phasen trennen sich in ca. 20 Sekunden. Die Dimensionierung eines kontinuierlichen technischen Reaktors wird bevorzugt so bemessen, daß das Reaktionsgemisch die Reaktionsendtemperatur im Reaktor erreicht.

Die nach der Reaktion auf dem Niveau der jeweiligen Reaktionsendtemperatur abgetrennte Säurephase ist farblos und klar und wird in der oben beschriebenen Weise aufkonzentriert, wobei die oben beschriebene Extraktion bzw. Reaktiv-Extraktion eingeschoben werden kann. Die so geführte Kreislaufsäure enthält nach dem erfindungsgemäßen Verfahren weniger als 25 ppm Salpetersäure und weniger als 25 ppm salpetrige Säure, z.B. je 5 bis 20 ppm sowie gegebenenfalls geringe Mengen an organischen Verunreinigungen.

## Beispiele

## Beispiel 1

In einem wärmeisolierten Sulfierbecher ($\varnothing$ 100 mm), versehen mit Stromstörern und 2 auf einer Welle sitzenden Turbinenrührern ($\varnothing$ 39,9 mm) wurden 41,3 g (0,367 mol) Chlorbenzol und 617,8 g $H_2SO_4$ (72 %ig) bei 110°C unter Rühren (1 800 U/min) vorgelegt (eingebrachte spezifische Rührleistung: 22 W/l) und in 1 bis 2 sek. mit einer auf 110°C erhitzten Mischung aus 32,3 g (0,33 mol) $HNO_3$ (65 %ig) und 50 g $H_2SO_4$ (72 %ig) versetzt, und man ließ ohne Kühlung reagieren. Nach ca. 75 sek. hatte das Reaktionsgemisch die Endtemperatur von 134°C erreicht und der Rührer wurde angehalten. Nach Phasentrennung (einige Sekunden) wurden 77,2 g organische Phase erhalten.

| Zusammensetzung (GC): | Chlorbenzol | 2,4 % |
|---|---|---|
| | 2-NCB | 38,4 % (NCB = Nitrochlorbenzol) |
| | 3-NCB | 1,1 % |
| | 4-NCB | 58,2 % |

Aus der Absäure ließen sich durch Extraktion mit Methylenchlorid 3,8 g Organika gewinnen.

| Zusammensetzung (GC): | Chlorbenzol | 0 % |
|---|---|---|
| | 2-NCB | 37,2 % |
| | 3-NCB | 0,6 % |
| | 4-NCB | 61,9 % |
| Gesamt-Ausbeute | 95,1 % der theoretischen Ausbeute | |

(fortgesetzt)

| Verhältnis p/o-NCB | 1,53. |
|---|---|

Die Extraktion diente in diesem kleinen Ansatz zur vollständigen Bestimmung der Ausbeute; sie entfällt in einem technischen Verfahren.

### Beispiel 2

In einem wärmeisolierten Sulfierbecher wie in Beispiel 1 wurden 53,9 g (0,367 mol) o-Dichlorbenzol und 637,2 g $H_2SO_4$ (78 %ig) bei 110°C unter Rühren (1 800 U/min) vorgelegt (eingebrachte spezifische Rührleistung: 22 W/l) und in 1 bis 2 sek. mit einer auf 110°C erhitzten Mischung aus 32,3 g (0,333 mol) $HNO_3$ (65 %ig) und 50 g $H_2SO_4$ (72 %ig) versetzt, und man ließ ohne Kühlung reagieren. Nach ca. 95 sek. hatte das Reaktionsgemisch die Endtemperatur von 135°C erreicht und der Rührer wurde angehalten. Nach Phasentrennung (einige Sekunden) wurden 65,2 g organische Phase erhalten.

| Zusammensetzung (GC): | o-Dichlorbenzol | 9,5 % |
|---|---|---|
| | 3,4-Dichlornitrobenzol | 75,8 % |
| | 2,3-Dichlornitrobenzol | 14,8 % |

Aus der Absäure ließen sich durch Extraktion mit Methylenchlorid 1,9 g Organika gewinnen.

| Zusammensetzung (GC): | o-Dichlorbenzol | 0,7 % |
|---|---|---|
| | 3,4-Dichlornitrobenzol | 79,4 % |
| | 2,3-Dichlornitrobenzol | 12,9 % |
| Gesamt-Ausbeute: | 96,0 % der theoretischen Ausbeute. | |

### Patentansprüche

**1.** Verfahren zur kontinuierlichen Herstellung von Mononitrohalogenbenzolen durch Umsetzung von Halogenbenzolen mit einem $HNO_3/H_2SO_4/H_2O$-Gemisch unter Bildung im wesentlichen der Mononitrohalogenbenzole und Reaktionswasser, gekennzeichnet durch die Schritte

a) Einspeisung der Reaktionsteilnehmer Halogenbenzol, $HNO_3$, $H_2SO_4$ und $H_2O$ in beliebiger Reihenfolge in einen mit Mischungsorganen ausgestatteten Reaktor, wobei

a1) die Menge an $HNO_3$ 1 bis 8 Gew.-%, die Menge an $H_2SO_4$ 56,5 bis 84,5 Gew.-% und die Menge an $H_2O$ den Rest zu 100 Gew.-% beträgt und 100 Gew.-% die Summe von $HNO_3 + H_2SO_4 + H_2O$ darstellen,

a2) das $H_2O$ als solches, als Verdünnungs-$H_2O$ der $HNO_3$, als Verdünnungs-$H_2O$ der $H_2SO_4$ oder in mehreren der genannten Formen eingesetzt wird, und

a3) das molare Verhältnis von Halogenbenzol zu $HNO_3$ 0,9 bis 1,5 beträgt,

b) rasche und intensive Vermischung der Gesamtmenge der Reaktionsteilnehmer, wozu eine Mischenergie von 1 bis 40 Watt pro Liter des gesamten Reaktionsgemisches, bevorzugt 3 bis 30 W/l, angewandt wird,

c) Durchführung der Umsetzung unter adiabatische Bedingungen in Reaktoren, die die Rückvermischung der Reaktionsteilnehmer weitgehend verhindern und in denen die Reaktionsteilnehmer beim Durchströmen mindestens 2mal redispergiert werden, wobei die Reaktionsteilnehmer mit solchen Temperaturen eingespeist werden, daß die Vermischung im Bereich von 60 bis 160°C erfolgt und die Temperatur am Ende der Reaktion 180°C nicht übersteigt,

d) Trennung des Reaktionsgemisches nach Durchführung der Reaktion in eine organische und eine anorganische Phase und

e) destillative Aufarbeitung der weitgehend $HNO_3$-freien anorganischen Phase unter Entfernung von Wasser.

2. Verfahren nach Anspruch 1, dadurch gekennzeichnet, daß die Reaktionsteilnehmer vor Eintritt in den die Rückvermischung weitgehend verhindernden Reaktor mit Hilfe von Mischorganen innerhalb einer Zeit von weniger als 3 sek. intensiv gemischt werden.

3. Verfahren nach Anspruch 1, dadurch gekennzeichnet, daß im Schritt e) die infolge der adiabatischen Reaktionsführung von der anorganischen Phase aufgenommene Reaktionswärme zur destillativen Ausschleusung von Wasser unter einem Druck von 40 bis 150 mbar, bevorzugt 40 bis 120 mbar, besonders bevorzugt 50-100 mbar, ausgenutzt wird und bevorzugt dabei die Temperatur und Konzentration der ablaufenden $H_2SO_4$ so eingestellt werden, daß diese ablaufende $H_2SO_4$ unmittelbar zum Einsatz in Schritt a) geeignet ist.

4. Verfahren gemäß Anspruch 1, dadurch gekennzeichnet, daß die Vermischung im Bereich von 70 bis 140°C, bevorzugt 80 bis 120°C erfolgt.

5. Verfahren gemäß Anspruch 1, dadurch gekennzeichnet, daß der Schwefelsäuregehalt in der Säurephase nach dem Schritt d) 60 bis 85 Gew.-%, bevorzugt 68 bis 80 Gew.-%, besonders bevorzugt 70 bis 78 % beträgt.

6. Verfahren gemäß Anspruch 1, dadurch gekennzeichnet, daß der Gehalt an zugesetzter Salpetersäure im Reaktionsgemisch zum Zeitpunkt der Vermischung, bezogen auf die Summe von Salpetersäure, Schwefelsäure und Wasser, 2 bis 6 Gew.-%, bevorzugt 2,5 bis 5 Gew.-% beträgt.

7. Verfahren gemäß Anspruch 1, dadurch gekennzeichnet, daß der Gehalt an Schwefelsäure im Reaktionsgemisch zum Zeitpunkt der Vermischung, bezogen auf die Summe von Salpetersäure, Schwefelsäure und Wasser, 65 bis 79 Gew.-%, bevorzugt 67,5 bis 77 Gew.-% beträgt.

8. Verfahren gemäß Anspruch 1, dadurch gekennzeichnet, daß das Molverhältnis von Toluol zu Salpetersäure 1,0 bis 1,5, bevorzugt 1,01 bis 1,3, besonders bevorzugt 1,05 bis 1,2 beträgt.

**Claims**

1. Process for the continuous preparation of mononitrohalogenobenzenes by reaction of halogenobenzenes with an $HNO_3/H_2SO_4/H_2O$ mixture with formation, essentially, of the mononitrohalogenobenzenes and water of reaction, characterized by the steps of

a) feeding the reactants halogenobenzene, $HNO_3$, $H_2SO_4$ and $H_2O$ in any sequence into a reactor equipped with mixing elements, where

a1) the quantity of $HNO_3$ is from 1 to 8% by weight, the quantity of $H_2SO_4$ is from 56.5 to 84.5% by weight and the quantity of $H_2O$ is the remainder to 100% by weight, and 100% by weight represents the sum of $HNO_3 + H_2SO_4 + H_2O$,

a2) the $H_2O$ is employed as such, as dilution $H_2O$ of the $HNO_3$, as dilution $H_2O$ of the $H_2SO_4$ or in a plurality of the forms mentioned, and

a3) the molar ratio of halogenobenzene to $HNO_3$ is from 0.9 to 1.5,

b) rapidly and intensively mixing the total quantity of the reactants, for which a mixing energy of from 1 to 40 watts per litre of the overall reaction mixture, preferably from 3 to 30 W/l, is employed,

c) carrying out the reaction under adiabatic conditions in reactors which substantially prevent the back-mixing of the reactants and in which the reactants are redispersed at least twice while flowing through the reactors, the reactants being fed in at temperatures such that the mixing takes place in the range from 60 to 160°C and the temperature at the end of the reaction does not exceed 180°C.

d) separating the reaction mixture, after carrying out the reaction, into an organic and an inorganic phase, and

e) working up the substantially $HNO_3$-free inorganic phase by distillation with removal of water.

2. Process according to Claim 1, characterized in that the reactants are intensively mixed using mixing elements for a period of less than 3 sec before entry into the reactor which substantially prevents back-mixing.

3. Process according to Claim 1, characterized in that in step e) the heat of reaction absorbed by the inorganic phase as a result of the adiabatic procedure is utilized for the distillative expulsion of water under a pressure of from 40 to 150 mbar, preferably from 40 to 120 mbar and particularly preferably 50-100 mbar, the temperature and concentration of the outflowing $H_2SO_4$ in this procedure preferably being adjusted such that this outflowing $H_2SO_4$ is suitable directly for use in step a).

4. Process according to Claim 1, characterized in that the mixing is carried out in the range from 70 to 140°C, preferably from 80 to 120°C.

5. Process according to Claim 1, characterized in that the content of sulphuric acid in the acid phase after step d) is from 60 to 85% by weight, preferably from 68 to 80% by weight and particularly preferably from 70 to 78%.

6. Process according to Claim 1, characterized in that the content of added nitric acid in the reaction mixture at the time of mixing, relative to the sum of nitric acid, sulphuric acid and water, is from 2 to 6% by weight, preferably from 2.5 to 5% by weight.

7. Process according to Claim 1, characterized in that the content of sulphuric acid in the reaction mixture at the time of mixing, relative to the sum of nitric acid sulphuric acid and water is from 65 to 79% by weight, preferably from 67.5 to 77% by weight.

8. Process according to Claim 1, characterized in that the molar ratio of toluene to nitric acid is from 1.0 to 1.5 preferably from 1.01 to 1.3 and particularly preferably from 1.05 to 1.2.

## Revendications

1. Procédé pour la préparation continue de mononitrohalogénobenzènes par réaction d'halogénobenzènes avec un mélange $HNO_3/H_2SO_4/H_2O$ avec formation essentiellement de mononitrohalogénobenzènes et d'eau de réaction, caractérisé en ce qu'il comporte les stades opératoires suivants :

a) introduction des réactifs : halogénobenzène, $HNO_3$, $H_2SO_4$ et $H_2O$ dans un ordre quelconque, dans un réacteur équipé d'organes de mélange, dans les conditions suivantes :

a1) la quantité de $HNO_3$ est de 1 à 8 % en poids, la quantité de $H_2SO_4$ de 56,5 à 84,5 % en poids et la quantité d'$H_2O$ représente le solde à 100 % en poids, la somme de $HNO_3$ + $H_2SO_4$ + $H_2O$ représentant 100 % en poids,
a2) l'eau est mise en oeuvre à l'état pur, à l'état d'eau de dilution de l'acide nitrique, à l'état d'eau de dilution de l'acide sulfurique ou dans plusieurs de ces formes, et
a3) le rapport molaire halogénobenzène/$HNO_3$ va de 0,9 à 1,5,

b) mélange rapide et intensif de la totalité des réactifs, avec application d'une énergie de mélange de 1 à 40 watts par litre du mélange de réaction total, de préférence de 3 à 30 W/l,
c) réalisation de la réaction dans des conditions adiabatiques dans des réacteurs empêchant le mélange en retour des réactifs et dans lesquels les réactifs, au cours de leur écoulement, sont redispersés au moins deux fois, les réactifs étant introduits à des températures telles que le mélange se produise dans l'intervalle de 60 à 160°C et que la température en fin de réaction ne dépasse pas 180°C,
d) séparation du mélange de réaction, après la réaction, en une phase organique et une phase inorganique et
e) traitement par distillation de la phase inorganique pratiquement exempte de $HNO_3$, avec élimination de l'eau.

2. Procédé selon la revendication 1, caractérisé en ce que les réactifs sont soumis à mélange intensif en une durée inférieure à 3 secondes à l'aide d'organes de mélange avant entrée dans le réacteur empêchant pratiquement le mélange en retour.

3. Procédé selon la revendication 1, caractérisé en ce que, au stade opératoire e), la chaleur de réaction absorbée par la phase inorganique en raison de la conduite adiabatique de la réaction est exploitée pour l'élimination de l'eau par distillation sous une pression de 40 à 150 mbar, de préférence de 40 à 120 mbar et plus spécialement de 50 à 100 mbar, avec de préférence réglage de l'acide sulfurique sortant à une température et une concentration permettant l'envoi direct de cet acide sulfurique au stade opératoire a).

4. Procédé selon la revendication 1, caractérisé en ce que le mélange est réalisé dans l'intervalle de 70 à 140°C, de préférence de 80 à 120°C.

5. Procédé selon la revendication 1, caractérisé en ce que, dans la phase acide, et après le stade opératoire d), la teneur en acide sulfurique est de 60 à 85 % en poids, de préférence de 68 à 80 % en poids et plus spécialement de 70 à 78 %.

6. Procédé selon la revendication 1, caractérisé en ce que la teneur du mélange de réaction en acide nitrique ajouté au moment du mélange est de 2 à 6 % en poids, de préférence de 2,5 à 5 % en poids par rapport à la somme de l'acide nitrique, de l'acide sulfurique et de l'eau.

7. Procédé selon la revendication 1, caractérisé en ce que la teneur en acide sulfurique du mélange de réaction au moment du mélange est de 65 à 79 % en poids, de préférence de 67,5 à 77 % en poids par rapport à la somme de l'acide nitrique, de l'acide sulfurique et de l'eau.

8. Procédé selon la revendication 1, caractérisé en ce que le rapport toluène/acide nitrique est de 1,0 à 1,5, de préférence de 1,01 à 1,3 et plus spécialement de 1,05 à 1,2.